# EUROPEAN PATENT APPLICATION

(11) **EP 1 779 871 A2**
(43) Date of publication of application: **02.05.2007**
(21) Application number: 06020382.5
(22) Date of filing: 28.09.2006
(51) Int. Cl.: A61L 9/12, A61L 9/03, A01M 1/20

(54) **Diffusing device for volatile substances**

(30) Priority: 25.10.2005 IT MI20052023
(71) Applicant: Zobele Holding SpA, 38100 Trento (IT)
(72) Inventor: Sordo, Walter, 38050 Cognola (TN) (IT)
(74) Representative: Petruzziello, Aldo

(57) **Abstract**

A diffusing device for volatile substances such as insecticides, deodorants and the like is described, comprising a container designed to contain a volatile liquid, a wick partially inserted in the container (1) and partially disposed outside it, and an under-cap (3) interposed between the neck (6) of the container and the wick (2), to hold the latter in position, wherein means (4, 10); (4') are provided such as to prevent the wick (2) and or the under-cap (3) from coming out of the container (1) in the event of traction on the outer part of the wick (2).

## Description

The present invention refers to a diffusing device for volatile substances, such as insecticides, deodorants and the like.

Devices of this type normally comprise a small bottle containing a volatile liquid in which are dissolved the active principles to be diffused, a wick inserted at least partially into the bottle so as to be impregnated with said volatile liquid, and possibly a heating means, for example of the electric type, or ventilation means, to promote diffusion of the volatile substances.

The wick is inserted into the container or small bottle through a so-called under-cap, that is, a ring which is placed inside the neck of the container, A cap, for example a screw cap, is then applied to the container and is removed at the time of use of the diffusing device.

A drawback of devices of the prior art is that the wick tends to come out of the container if traction is applied on the outer part thereof, with the risk of leakage of the product, which can be dangerous because of the noxious nature of the substances used, especially if handled by children. Removal of the wick and subsequent reinsertion into the container can compromise working of the device, especially if attempts are made to re-fill the container with liquid, an operation which is not normally allowed.

Solutions have been proposed to make extraction of the wick from the container more difficult, but such solutions have not proved entirely satisfactory.

Thus, for example, US 6.236.807 proposes a pin to be inserted transversally to the wick, of such a length as to interfere with the under-cap, or even that the pin be inserted in the under-cap and in the wick, so as to render the two elements virtually integral with each other. With this solution extraction of the wick from the under-cap is prevented, but not extraction of the under-cap and wick assembly if strong traction is exerted on the outer part of the latter.

Object of the invention is precisely to overcome the above-mentioned drawback, that is, to make it impossible to extract the wick from the container of volatile liquid, alone or together with the under-cap, irrespective of the traction force exerted on the outer part of the wick.

This object is achieved according to the invention with the characteristics of appended independent claim 1.

Advantageous embodiments of the invention are apparent from the dependent claims.

According to a first embodiment of the invention, the wick has, in the part thereof disposed inside the liquid container, an enlargement, in a single body or added which, in the event of traction on the wick, interferes with the inner hole of the under-cap, preventing it from coming out. At the same time this enlargement, which has a conical or flared shape, widens some elastic tongues provided beneath the under-cap, making them interfere with the neck of the container, thus preventing any possibility of the under-cap being expelled from the container. The greater the force of the traction exerted on the wick, the greater the deformation of the tongues, and thus the greater the interference of the under-cap with the neck of the container.

In a second embodiment, a stiff ring, preferably of metal flattened in one of its two transverse dimensions, is disposed around the wick. During insertion of the wick into the neck of the container, the ring is disposed inclined with respect to the axis of the wick, which is inserted into the container in a slightly out-of-axis position, so as to allow the ring to be inserted also. Once the ring has passed the neck of the container, the under-cap is inserted, disposing the wick in axis with the container. At this point if traction is exerted outward on the wick, it will produce straightening of the ring which, having a transverse dimension greater than the inside diameter of the neck of the container, is disposed in abutment against the latter, without the possibility of exiting therefrom. Therefore traction, however strong, on the outer part of the wick prevents extraction thereof.

Further characteristics of the invention will be made clearer by the detailed description that follows, referring to purely exemplary and therefore non limiting embodiments of the invention, illustrated in the appended drawings, in which:
- Figures 1A-1D are median sectional views illustrating successive stages in assembly of the wick in the container for volatile liquids of a diffusing device according to a first embodiment of the invention;
- Figure 2 is a median sectional view only of the under-cap shown in Figures 1A-1D;
- Figure 3 is a perspective view of the under-cap shown in an upside-down position with respect to that of Figure 2;
- Figure 4 is an exploded view of the various component parts of a container for volatile liquid with wick of a diffusing device according to a second embodiment of the invention;
- Figures 5A-5E illustrate successive stages in insertion of the wick into the container according to the embodiment of Figure 4;
- Figures 5F-5I illustrate successive stages in the event of attempted removal of the wick from the container of Figure 4.

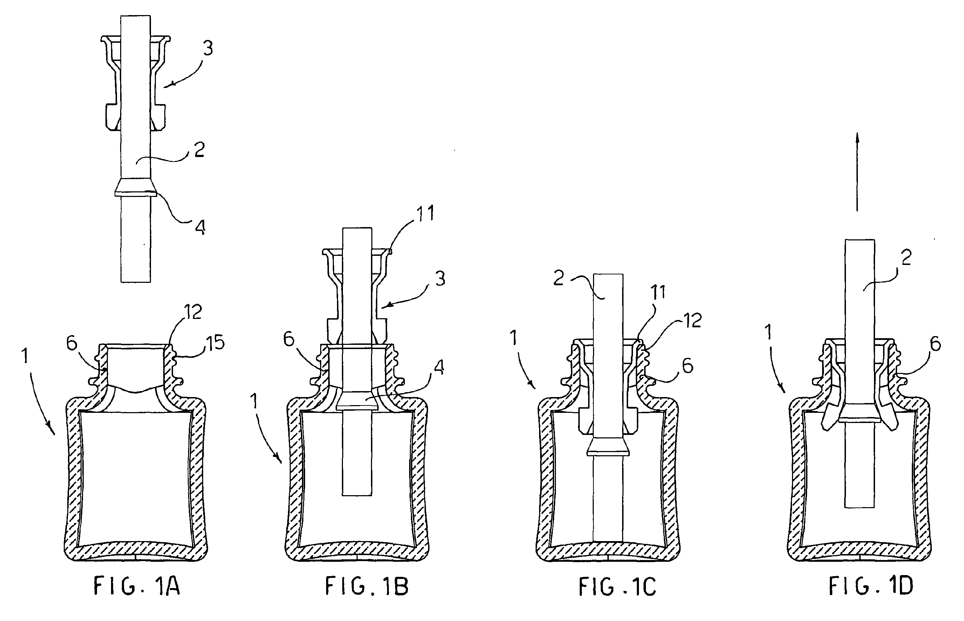

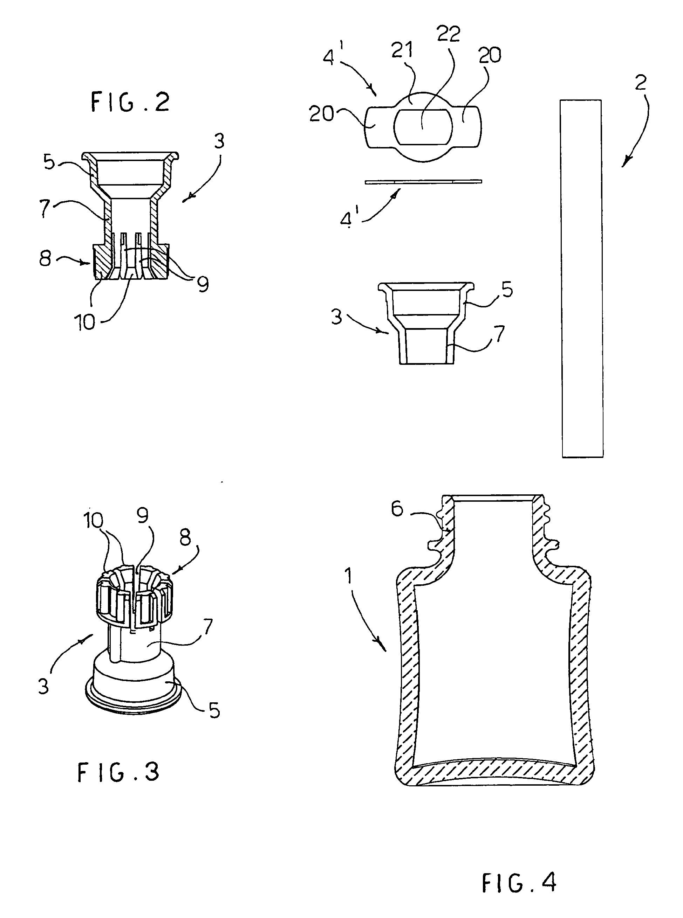

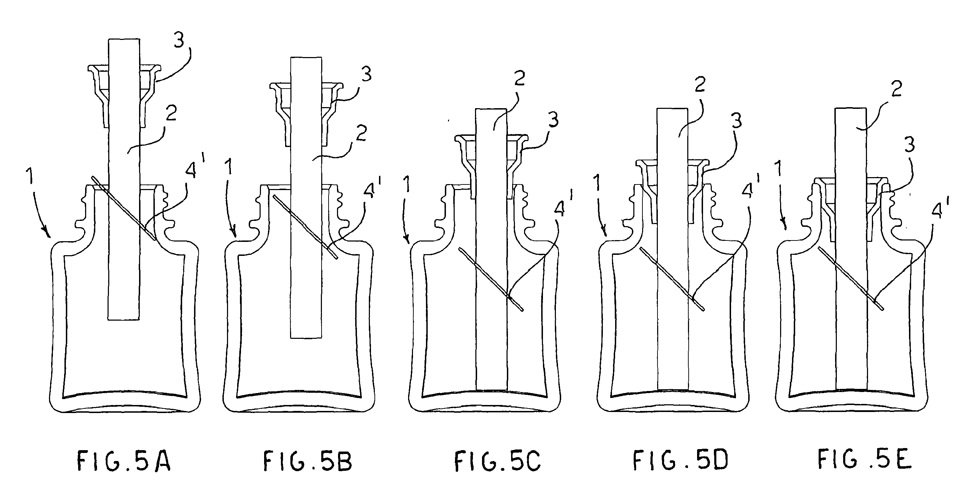

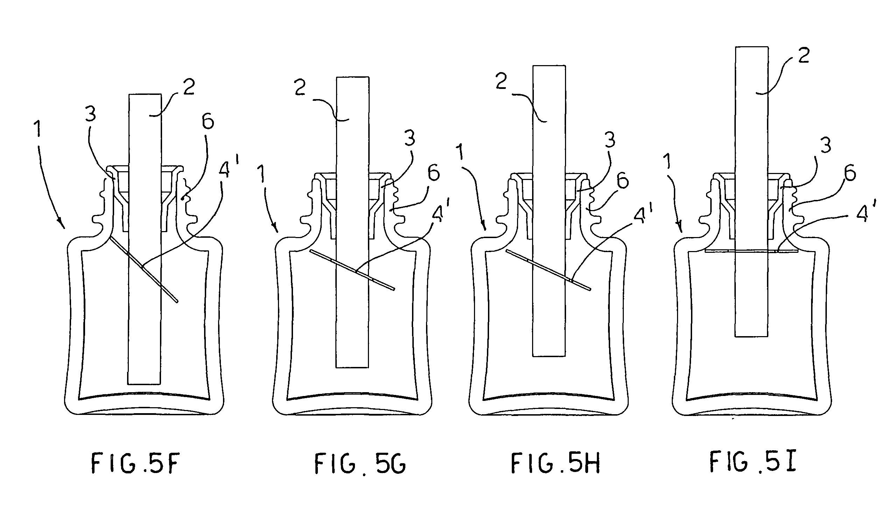

With reference initially to Figures 1A-1D, 2 e 3, the first embodiment of the invention is described; the same reference numerals will be used for the second embodiment illustrated in Figures 4, 5A-5I, to denote like or corresponding parts.

In Figures 1A-1D, (1) denotes a container or bottle destined to contain a volatile liquid, such as insecticide, deodorant and the like, (2) denotes a wick designed to be inserted at least partially into the container (1) and (3) an under-cap, or an annular body destined to be interposed between the neck of the container and the wick, to hold the latter in position.

According to the invention, the wick (2), in an intermediate portion thereof, designed to be disposed inside the container (1), has an enlargement (4), frustoconical or upwardly flared in shape. This enlargement is advantageously formed in a single body with the wick, but could also be added thereon.

The under-cap (3) has a hollow cylindrical shape, with an upper portion (5) having a diameter substantially corresponding to the inside diameter of the neck (6) of the container (1), joined to an intermediate portion (7) with a smaller diameter, ending at the bottom with a thicker portion (8), which thus causes an increase in diameter with respect to the intermediate portion (7).

A plurality of axial slots (9), such as to give rise to a plurality of flexible tongues 10 with a flared inner mouth is provided on the lower portion (8) and on part of the intermediate portion (7).

With reference to Figures 1A-1D the method of insertion of the wick into the container and operation of the assembly that prevents extraction of the wick and of the under-cap from the container will now be described.

As can be seen in Figure 1A, the wick is inserted from beneath into the under-cap (3), and the wick and under-cap assembly is inserted into the container (1) (Figures 1B-1C), until an upper annular ridge (11) of the under-cap (3) abuts on the upper edge (12) of the neck (6) of the container (1) (Figure 1C).

If, in such a condition of use, traction is exerted on the wick (2), as indicated by the arrow in Figure 1D, the wick slides in the under-cap (3) until the enlargement (4) abuts against the lower edge of the terminal portion (8) of the under-cap. At this point, further traction on the wick (2) causes widening, through elastic yielding, of the tongues (10) which interfere against the base of the neck (6) of the container (1). In this manner the under-cap (3) remains jammed in the neck of the container (1) and the wick (2) remains jammed in the under-cap (3), so that the wick and/or the under-cap are prevented from coming out of the container, whatever the traction force exerted on the wick (2). Indeed, the greater the force, the more the wick and under-cap become jammed in the neck of the container.

The intended object of the invention is thus fully achieved.

In the example illustrated, a thread (15) is provided on the outside of the neck (6) of the container, with which engages a cap for covering the wick (2), not shown in the figures. The same thread (15) can be used to assemble the container (1) with the wick (2), for example in an electric heating device to promote diffusion of the volatile substances. This electrical diffuser has not been illustrated because it does not form the subject matter of the invention.

With reference now to Figures 4, 5A-5I a second embodiment of the invention is illustrated.

In this second embodiment, the wick (2) is of the traditional type and cylindrical in shape, in that it does not have the enlargement (4), and the under-cap (3) also is a substantially conventional shape, that is, without the flexible lower tongues (10) provided in the preceding embodiment.

Instead there is a ring (4') having an elongated shape in a plan view, that is, such that the dimension in a transverse direction is smaller than the inside diameter of the neck (6) of the container (1) and the size in the other transverse direction is greater than the inside diameter of the neck (6). In practice, as shown in Figure 4, the ring (4') has a substantially circular central portion (21) and two opposite protrusions (20) which cause the lengthening of the ring.

The ring (4'), preferably of metal, has centrally a slot-shaped hole (22) having an elongated shape in the same direction as the lengthening of the ring, such that its size in said direction is greater than the diameter of the wick (2), whilst in the direction at right angles thereto it is smaller than said diameter.

In this manner, the ring (4') can be fitted with a certain interference on the wick (2), remaining locked in the desired position, but with the possibility of oscillation in the direction of the largest dimension of the ring.

With reference to Figures 5A-51 insertion of the wick into the container and operation of the ring which prevents it from coming out will now be described.

The ring (4') is fitted on the wick (2) and disposed in the position of greatest inclination (Fig.5A). At the same time the wick (2) is inserted into the under-cap (3).

After the lower edge of the ring (4') has passed the base of the neck (6) of the container, the wick is slightly out of axis with respect to the container (Figure 5B) to facilitate its further insertion (Figure 5C). The under-cap (3) is subsequently disposed on the neck of the container, causing the wick to be centred with respect to the container (Figure 5D). Lastly, the under-cap is inserted into the neck of the container (Figure 5).

In this condition, the upper edge of the ring (4'), which remains in an inclined position, is disposed outside the plan-view projection of the inside circumference of the neck (6) of the container. Therefore, upward traction of the wick (2) (Figure 5F) causes interference of said edge with the base of the neck of the container and subsequent straightening of the ring (4') (Figures 5G, 5H, 51) which is disposed in abutment against the base of the neck of the container, preventing extraction of the wick therefrom.

Therefore, this embodiment too achieves the intended object of avoiding extraction of the wick and/or of the under-cap from the container (1).

Of course, the invention is not limited to the particular embodiments described above and illustrated in the appended drawings, but numerous modifications of detail within the reach of a person skilled in the art can be made thereto without thereby departing from the scope of the invention as set forth in the appended claims.

## Claims

1. A diffusing device for volatile substances, such as insecticides, deodorants and the like, comprising a container (1) designed to contain a volatile liquid, a wick (2) partially inserted in said container (1) and partially disposed outside the container, and an under-cap (3), with an annular shape, interposed between the neck (6) of the container and the wick (2) to maintain the latter in position, as well as a possible wick-covering cap, **characterized in that** it provides means (4, 10; 4') able to prevent extraction of the wick (2) and/or of the under-cap from the container (1) in the event of traction on the outer part of the wick (2).

2. A device according to claim 1, **characterized in that** said means (4,10) designed to prevent removal of the wick and/or under-cap, consist of an enlargement (4) of the wick (2) situated in the part thereof disposed inside the container, and of a plurality of flexible tongues (10) provided in the bottom part of the under-cap (3), such that outward traction on the wick (2) causes jamming of the enlargement (4) of the wick (2) in the under-cap (3) and simultaneous widening, through elastic yielding, of said tongues (10) with consequent jamming of the under-cap (3) in the neck (6) of the container (1).

3. A device according to claim 2, **characterized in that** said enlargement (4) is formed in a single body with the wick (2) or in a part applied thereon.

4. A device according to claim 2, **characterized in that** said enlargement (4) is frustoconical in shape or flared towards the top and said flexible tongues (10) of the under-cap (3) have a complementary conical entry guide.

5. A device according to claim 2, wherein said flexible tongues (10) are determined by axial slots (9) made in the bottom part of the skirt of said annular under-cap (3).

6. A device according to claim 1, **characterized in that** said means (4') designed to avoid extraction of the wick (2) and/or of the under-cap (3) from the container (1) comprise a ring (4') fitted interferingly on the wick (2), with the possibility of oscillation around an axis perpendicular to the axis of the wick, said ring (4') having in a plan view one transverse dimension smaller than inside diameter of the neck (6) of the container and the other transverse dimension greater than said inside diameter, so that the ring (4'), disposed in an inclined position on the wick (2) can pass beyond the neck (6) of the container (1) when the wick is inserted therein in an out-of-axis position, and dispose itself in a position interfering with the neck of the container when the wick is centred following assembly of the under-cap (3), such that outward traction on the wick (2) causes the ring (4') to push against the neck of the container and to straighten, with consequent positioning in abutment against the base of the neck (6) of the container, so as to prevent the wick and/or under-cap from coming out.

7. A device according to claim 6, **characterized in that** said ring (4') has an oblong central hole, the transverse dimension of which is smaller than the diameter of the wick, so as to allow assembly with interference on the latter and simultaneous oscillation in the other transverse dimension.

8. A device according to claim 6, **characterized in that** said ring (4') has in a plan view a substantially circular central part, of a smaller size than the inside diameter of the neck (6) of the container and two opposite extensions (20) such as to determine a greater size of the ring in this direction than the inside diameter (6) of the container.

9. A device according to claim 6, wherein said ring (4') is of rigid material, particularly metal.
